# EUROPEAN PATENT APPLICATION

(11) **EP 1 978 093 A1**
(43) Date of publication of application: **08.10.2008**
(21) Application number: 07105672.5
(22) Date of filing: 04.04.2007
(51) Int. Cl.: C12N 9/10, C12N 15/62, G01N 33/535

(54) **Method of crosslinking two objects of interest**

(71) Applicant: Ecole Polytechnique Fédérale de Lausanne (EPFL), 1015 Lausanne (CH)
(72) Inventor: Johnsson, Kai, 1006 Lausanne (CH)
(74) Representative: Wilming, Martin

(57) **Abstract**

The invention provides a method of crosslinking two objects of interest, comprising the steps of: i) providing a fusion protein comprising at least a first protein and a second protein, wherein both the first and the second protein are, based on their structure and function, capable of forming a covalent bond with given substrates, and which first and second proteins are of substantially non-overlapping substrate selectivity, preferably of different substrate specificity; ii) providing a first object of interest, comprising a substrate moiety for the first protein of the said fusion protein, and providing a second object of interest, comprising a substrate moiety for the second protein of the said fusion protein; and iii) reacting said first protein of the fusion protein with the substrate moiety of said first object, and reacting said second protein of the fusion protein with the substrate moiety of said second object, thereby covalently crosslinking the first object to the second object via the said fusion protein. Most prominent applications of the disclosed method are, due to the straight-forward, reliable, directional and fast crosslinking reactions: the derivatization of cells, antibodies and the crosslinking of proteins.

## Description

### FIELD OF INVENTION

In broad sense, the present invention relates to methods of covalently crosslinking two objects of interest in a reliable, selective and directional manner, mediated by a protein, especially a fusion protein. In particular, the invention concerns methods for use in derivatization of biological molecules, e.g. proteins, antibodies, viruses or cells, to methods of crosslinking of object proteins, methods to directionally couple physical or chemical objects for applications in nanotechnology and to methods of binding of a molecular object of interest to a solid support.

### BACKGROUND OF THE INVENTION

The specific connection or crosslinking of two or multiple objects which may be (i) biomolecules such as proteins and DNA, (ii) cells and viruses, as well as (iii) nanomaterials and synthetic molecules, is an omnipresent issue in biotechnology and nanotechnology. One way to achieve the connection of objects is through the mediation of proteins. In this case the protein must possess an affinity for the two objects. This affinity can be the natural affinity of the protein towards the object, such as the affinity of antibodies towards their antigens. As the antibody has two binding sites, it can connect two antigens in a non-covalent manner. Alternatively, the objects might be derivatized with ligands that are specifically recognized by some protein. The best know example for such a protein-ligand pair is streptavidin. Two different objects can be biotinylated and then connected through the tetrameric streptavidin or avidin, which possesses four different binding sites. Such biotinylated objects can be cells, proteins, physical objects or synthetic molecules. The biotinylation can be achieved through a simple coupling of an activated biotin derivative, such as a commercially activated ester, or through the action of an protein. The addition of streptavidin to such biotinylated objects then leads to the connection mediated by the tetrameric streptavidin. This and related approaches suffer from two main points: Firstly, the connection is not covalent and irreversible and thus is not stable under conditions in which the protein that mediates the connection is denatured. Such conditions can be high temperature or organic solvents. Secondly, the connection/crosslinking is not directional when multiple objects are present. For example, addition of streptavidin to two different biotinylated proteins A and B will lead to the formation of a number of different complexes (i.e. crosslinking of A with A, A with B, B with A, and higher aggregates), their relative frequency determined by the relative concentrations of the biotinylated proteins and streptavidin.

An example for the importance of connecting different objects are antibody-based bioassays. Antibodies (the first object) are the key element in numerous bioassays and bind non-covalently to a specific object of interest. In general, they need to be derivatized with a probe (the second object) that allows for detection of the antibody. Such probes can be proteins, fluorophores, gold particles etc. The probe is attached to the antibody either through direct chemical coupling or the probe is coupled to a secondary antibody that specifically recognizes and binds to the primary antibody. In any case, chemical derivatization of an antibody with a probe is a prerequisite for this technique, and for each different experiment a differently labelled antibody needs to be prepared or purchased.

On the other hand, the transfer of a label from substrates to fusion proteins consisting of an O⁶-alkylguanine-DNA alkyltransferase (hereinafter: AGT) and a protein of interest is known inter alia from WO 02/083937 (PCT/GB02/01636), WO 2004/031404 (PCT/EP03/10859), WO 2004/031405 (PCT/EP03/10889) and WO 2005/085431 (PCT/EP2005/050889), respectively; the disclosure of these documents is incorporated by reference into the present application. In these documents, AGT is fused to a protein of interest, and the AGT is used to covalently attach a label to the fusion protein which subsequently allows for detection and/or manipulation (e.g. purification or immobilization) of the fusion protein. Most recently, the fusion protein ^{M}AGT-DEVD-^{L}AGT has been labelled with two different fluorophores and an intramolecular FRET has been detected (Heinis et al., ACS Chemical Biology 1(9), 2006, A-J).

Recently, mutants of AGT (hereinafter ACTs) were developed (Patent application number EP06117779, entitled "Labelling of fusion proteins with synthetic probes", incorporated herein by reference) that specifically react with benzylcytosine (hereinafter BC) derivatives and which allow the labelling of ACT fusion proteins using BC derivatives the same way as AGT fusion proteins can be labelled with benzylguanine (hereinafter BG) derivatives. Importantly, the reactivity of ACT versus BG derivatives is below 1% of the reactivity versus BC derivatives and the reactivity of AGT versus BC derivatives is 1% below the reactivity of AGT versus BG. ACT and AGT thus have substantially non-overlapping substrate specificity.

Yet another approach of covalently attaching a label to a protein is the HaloTag™ (Promega Corporation, 2800 Woods Hollow Road, Madison, WI, USA), described in Los et al., Cell Notes 11, 2005, 2-6, WO 2006/093529 and WO 2004/072232. The system is based on a genetically engineered hydrolase, in which the final hydrolysis step is impaired and the label thus remains covalently attached to the hydrolase.

Concluding, the HaloTag™, the AGT system and the ACT system are known in the art for covalently attaching a label (i.e. a tag which provides a possibility of detection or further manipulation, but which label is not of interest by itself) to a fusion protein comprising a protein of interest and the HaloTag™ or AGT, respectively.

Concluding, no method of covalently crosslinking two objects of interest in a reliable, selective and directional manner is currently available.

It is thus an object of the invention to overcome the above-mentioned drawbacks of prior art methods of crosslinking/connecting two objects of interest, i.e. to provide a method which works reliably, selectively and directionally, and especially to provide a method of crosslinking two objects of interest for use in derivatization of e.g. antibodies, viruses or cells, methods of crosslinking of physical and chemical objects on the nanometer scale and methods of binding of an object of interest to a solid support or a cell.

### SUMMARY OF THE INVENTION

This invention provides a fusion protein comprising at least a first protein and a second protein, wherein both the first and the second protein are, based on their structure and function, capable of forming a covalent bond with given substrates, and which first and second proteins are of substantially non-overlapping substrate selectivity, preferably of different substrate specificity; with the provisio that the fusion protein is not ^{M}AGT-DEVD-^{L}AGT or ^{L}AGT-DEVD-^{M}AGT. (^{M}AGT-DEVD-^{L}AGT and ^{L}AGT-DEVD-^{M}AGT do not contain two proteins with different selectivity/specificity; thus, crosslinking would have to be carried out sequentially and could not be reliably achieved in mixtures of both substrates for ^{M}AGT and ^{L}AGT, respectively.)

As noted above, such fusion proteins are not known in the art, to the best of applicant's knowledge. Neither the AGT and ACT nor the HaloTag™ have not been reported in fusion proteins to allow for connecting two objects of interest to each other. As will be outlined below in any detail, these fusion proteins serve as reliable, selective and directional tools for dual, covalent crosslinking with different objects of interest (especially proteins), also allowing inter alia for novel approaches in derivatization of cells, viruses, antibodies and any object (biological or synthetic) that can be derivatized with the substrate reacting with the protein.

The invention moreover provides a recombinant DNA sequence encoding for the said fusion protein; an expression vector containing an expression cassette encoding for the said fusion protein; and a prokaryotic or eukaryotic host cell line, enabled to functionally express the said fusion protein and/or transformed with the said expression vector.

Yet another aspect of the invention concerns a kit-of-parts, comprising, besides the said fusion protein and/or the said expression vector and/or the said host cell line, at least one molecule comprising a substrate moiety for at least either the first or the second protein of the said fusion protein. This substrate can be a biomolecule, a cell, a virus or any other synthetic or natural object derivatized with the substrate to allow for the covalent and specific crosslinking/connecting of two objects.

As will be outlined below in any detail, the molecule comprising a substrate moiety is used for modifying the respective object of interest suchlike that this object of interest then presents a substrate moiety for the said fusion protein, thereby forming a covalent crosslink between the object of interest and the fusion protein. Preferably, the kit-of-parts encompasses at least two such molecules, the one molecule comprising a substrate moiety for the first protein of the said fusion protein, and the other molecule comprising a substrate moiety for the second protein of the said fusion protein.

Yet a further aspect of the present invention thus concerns a method of crosslinking two objects of interest, comprising the steps of:
i) providing a fusion protein comprising at least a first protein and a second protein, wherein both the first and the second proteins are, based on their structure and function, capable of forming a covalent bond with given substrates, and which first and second proteins are of different substrate selectivity, preferably of different substrate specificity;
ii) providing a first object of interest, comprising a substrate moiety for the first protein of the said fusion protein, and providing a second object of interest, comprising a substrate moiety for the second protein of the said fusion protein;
iii) reacting said first protein of the fusion protein with the substrate moiety of said first object, and reacting said second protein of the fusion protein with the substrate moiety of said second object, thereby covalently crosslinking the first object to the second object via the said fusion protein.
   Both covalent bonds can be formed simultaneously due to different substrate selectivity or specificity, i.e. no stepwise labelling is necessary. However, the crosslinking can also be achieved through sequential reactions; here, the order of the reactions is not relevant.

The present invention moreover provides a method of modifying a first object and/or a second object, for use with a fusion protein comprising at least a first protein and a second protein, wherein both the first and the second protein are, based on their mechanism of enzymatic catalysis, capable of forming a covalent bond with given substrates, and which first and second proteins are of different substrate selectivity, preferably of different substrate specificity, comprising the steps of:
i) transferring to the first object a substrate moiety for the first protein of the said fusion protein; and/or
ii) transferring to the second object a substrate moiety for the second protein of the said fusion protein.

### DESCRIPTION OF THE FIGURE

| | |
|---|---|
| SEQ ID NO:1 | ^{M}AGT-D^{E}VD-^{L}AGT |
| SEQ ID NO:2 | HaloTag^{™}-AGT |
| SEQ ID NO:3 | ACT1 |

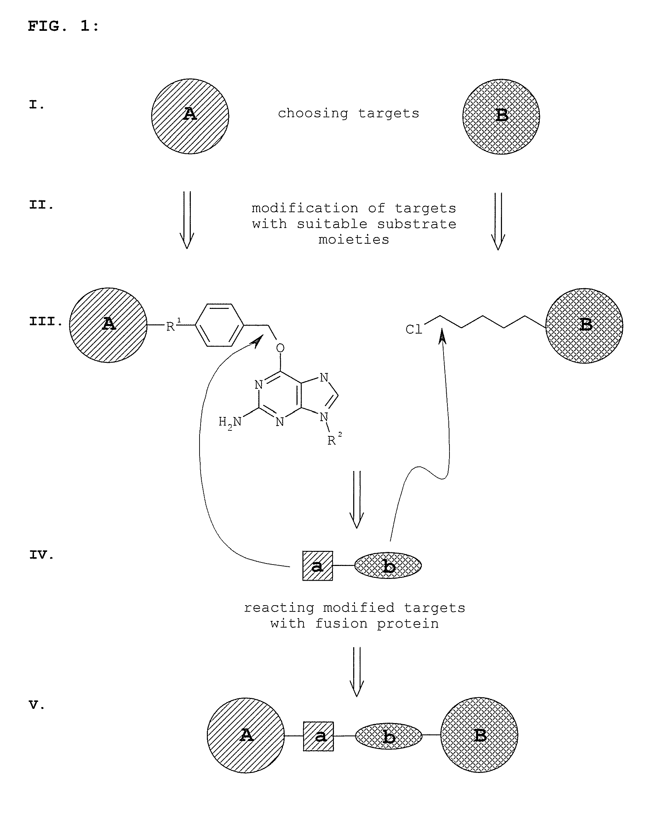

Fig. 1. An embodiment of the present invention. Two objects (A) and (B) are chosen in step (I.), such as e.g. cells, antibodies, solid surfaces, proteins, proteins, etc. In step (II.), these objects are modified with suitable substrate moieties, as will be outlined below in more detail. In step (III.), object (A) is modified in the present example with an O⁶-benzylguanine derivative which is a substrate for AGT. R² represents hydrogen, β-D-2'-deoxyribosyl or β-D-2'-deoxyribosyl which forms part of a deoxyribonucleotide, preferably having a length between 2 and 99 nucleotides; R¹ represents a linker group as commonly applied in the art, for example a flexible linker group such as a substituted or unsubstituted alkyl chain or a polyethylene glycol. Object (B) is, in the present example, modified with an aliphatic halogenated (here: chlorinated) alkyl chain; the modified object (B) is thus a substrate for the modified hydrolase of the Halo-Tag^{™}. In step (IV.), a fusion protein comprising AGT (a) and the HaloTAG^{™} (b) is reacted with both objects, each presenting substrates for either AGT or the HaloTag^{™}, respectively. As shown in step (IV.), a covalent crosslink is thereby formed between object (A) and object (B), mediated by the fusion protein of AGT and the HaloTag^{™}. As will be readily understood by the person of routine skill in the art, at least fusion proteins of AGT and ACT, or fusion proteins of ACT and the HaloTag^{™} are similarly applicable.

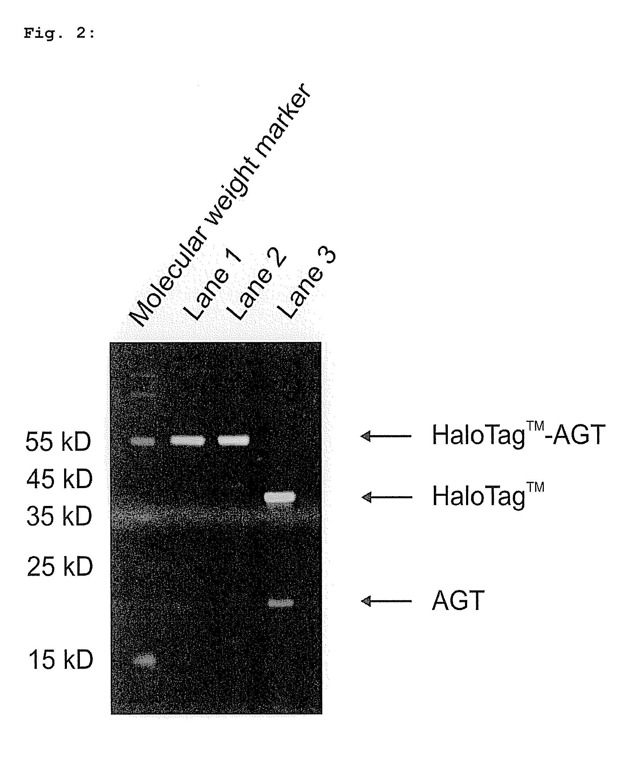

Fig. 2. Simultaneous labelling of a HaloTag^{™}-AGT (2 µM in PBS) fusion protein with two different fluorophores (simulating objects of interest) and analysis using SDS-PAGE and a laser-based fluorescence scanner (BioRad). Green color represents fluorescence resulting from fluorescein, red color represents fluorescence resulting from Cy3 and yellow color representing fluorescence from fluorescein and Cy3. Lane 1: Labelling of HaloTag^{™}-AGT (2 µM in PBS) with fluorescein (green) using commercially available diAcFAM (3 µM, Promega), the substrate for the Halo-Tag^{™}. Lane 2: Labelling of HaloTag^{™}-AGT (2 micromolar in PBS) with fluorescein (green) and Cy3 (red) using commercially available diAcFAM (3 µM, Promega), the substrate for the HaloTag^{™}, and BG-Cy3 (3 µM), the substrate of AGT; yellow colour demonstrates that the protein is labelled with both fluorophores. Lane 3: Digestion of the sample used for Lane 2 by addition of preScision^{™} Protease (0.4 units; GE Healthcare). The protease cleaves HaloTag™-AGT at the linker between the proteins, generating fluorescein-labelled HaloTag^{™} (about 40 kD, green) and Cy3-labeled AGT (about 20 kD, red). This control serves to verify the specificity of the labelling.

### DETAILED DESCRIPTION OF THE INVENTION

As briefly outlined above, the invention provides a fusion protein comprising at least a first protein and a second protein, wherein both the first and the second proteins are, based on their mechanism of enzymatic catalysis, capable of forming a covalent bond with given substrates, and which first and second proteins are of different substrate selectivity, preferably of different substrate specificity; with the provisio that the fusion protein is not ^{M}AGT-DEVD-^{L}AGT or ^{L}AGT-DEVD-^{M}AGT (disclosed in Heinis et al., ACS Chemical Biology 1(9), 2006, A-J, and further references therein; the disclosure of these documents is incorporated by reference into this application with respect to ^{L}AGT-DEVD-^{M}AGT and ^{L}AGT-DEVD-^{M}AGT).

The term "protein or polypeptide which, based on its structure and function, is capable of forming a covalent bond with a given substrate", or equivalent wording, is here and henceforth understood as follows: Polypeptides or proteins which possess i) at least one defined substrate binding region for the given substrate, and ii) at least one region, which allows for the irreversible transfer of (part of) the substrate which is bound in the substrate binding region onto an aminoacid residue of the protein. The respective protein or polypeptide possesses a reactivity for its substrate that allows its specific and covalent labelling in the presence of other proteins. Necessary reactivity is here defined as a rate for the covalent bond formation between the polypeptide and the given substrate that is at least 100 times, preferably 1'000 times, most preferably 1'000'000 times faster than the reaction of the reference proteins not possessing such a substrate-binding region such as bovine serum albumin (BSA), chymotrypsin and any of the 20 natural amino acids with this substrate. The respective proteins thus get permanently modified. The respective proteins are, due to their permanent modification, sometimes referred to as "suizymes". Examples of "suizymes" are ACT and AGT, which transfers an alkyl group from an alkylguanine in an S_{N}2 reaction onto one of its own cysteines, resulting in an irreversibly alkylated protein. Another example is the HaloTag^{™}, which is a genetically engineered hydrolase, in which the release of an intermediate by hydrolysis is impaired:
R-Cl + HaloTag^{™} → R-HaloTag^{™} + Cl⁻ -H₂O → no hydrolysis as in wildtype hydrolase (→ R-OH + HaloTag(TM) + H⁺ + Cl⁻).
A characteristic of all such "suizymes" is the nucleophilc displacement of a leaving group from the substrate by an aminoacid residue of the "suizyme".

These fusion proteins serve as useful tools in various applications by allowing for selective, preferably highly selective, most preferably specific and covalent crosslinking of two objects. Towards this end, only the desired objects need to be modified, either in vivo or in vitro, with substrate moieties for the respective protein of the fusion protein and be then reacted with the fusion protein, either in vivo or in vitro. The two reactions leading to covalent linkage of the two objects can be carried out stepwise or, even more preferably, simultaneously, even in complex mixtures.

It is to be noted that, dependent on the number of presented substrate moieties on the respective objects, various scenarios can be generated: in a first scenario, a 1:1 ratio of the crosslinked objects can be achieved by each of the objects presenting only one single substrate moiety. In yet a further scenario, ratios of 1:X with X > 1 can be easily achieved when one of the objects presents X substrate moieties. Moreover, complex networks can be generated when both the first and the second objects present more than one substrate moiety for the respective protein of the fusion protein. Of course, alternatively or additionally, the fusion protein may be provided with multiple copies of the first or the second protein, or even with yet further different protein(s) of different selectivity or specificity. A possible application for the connection of multiple copies of one object to one copy of the other object is the labelling of an antibody with multiple copies of the protein horseradish peroxidase. This would increase the sensitivity in peroxidase-based ELISA assays.

Concluding, the fusion proteins according to the present invention open up new horizons in various aspects:

First, antibodies can be more easily and flexibly derivatized than with the presently known approaches. The general techniques of antibody handling and derivatization are known in the art; e.g. from (Hermanson, G. T. "Bioconjuation techniques", Academic Press, San Diego, USA; 1996), incorporated by reference herein. In a straight-forward strategy according to the present invention, the antibody is first e.g. labelled with a substrate for one protein of the fusion protein (in case of AGT, e.g. with a benzylguanine derivative). For example, the antibody is incubated with BG carrying an activated ester group (i.e. N-hydroxysuccinimid esters; commercially available from Covalys Biosciences), leading to formation of stable amide bonds between the BG and lysine side chains and the aminotermini. Subsequently, the antibody only needs to be incubated with the corresponding fusion protein as outlined above, and with a second object which is similarly modified with a substrate moiety for the other protein of the fusion protein, respectively. As will now be evident to the person of routine skill in the art, one single modified antibody and one single fusion protein can be used for a vast variety of applications, depending only on the nature of the second object which is chosen. To mention only some of the possible applications, the second object can be e.g. other proteins, cells, solid surfaces, labels, viruses, quantum dots, any spectroscopic probes useful for imaging technologies in vivo such as MRI or PET, for fluorescence microscopy, radioactive probes, affinity probes such as biotin or digoxigenin, DNA or deoxyribo-oligonucleotides, RNA or ribo-oligonucleotides, antibodies, autofluorescent proteins. The coupling reactions between the fusion protein and their substrates are very fast (approx. 10⁴ sec⁻¹ M⁻¹) and can be performed due to their selectivity or preferably specificity even in complex mixtures, without the need for any purification steps, thus greatly facilitating the handling and increasing the flexibility of usage of a given antibody.

For the modification of cells (here the first object), the cells can be derivatized with a substrate for one of the fusion proteins e.g. by using an activated N-hydroxysuccinimid ester of said substrate using standard procedures such as described in the manual for the biotinylation of cells using an N-hydroxysuccinimid ester of biotin (*"Cell surface protein isolation Kit";* Pierce, part of Thermo Fischer Scientific). The modified cells are then simply incubated with the fusion protein and the second object.

According to preferred embodiments, the first and the second proteins are of orthogonal substrate specificity, i.e. the substrate for the first protein of the fusion protein is not at all a substrate for the second protein of the fusion protein, and vice versa (e.g., the combination of an AGT and the HaloTag^{™}); thus, the coupling of both objects to the fusion protein is fully directional, even in complex mixtures and even if carried out simultaneously. Consequently, the formation of homodimers can be reliably prevented. In any case, if the first and the second proteins are not of completely orthogonal substrate specificity, at least a substantially non-overlapping substrate selectivity is required, i.e. both proteins must not exhibit more than 5% reactivity against the substrate of the respective other protein, preferably not more than 3%, most preferably not more than 1%.

In currently preferred embodiments, one of the said proteins of the fusion protein is an O⁶-alkylguanine-DNA alkyltransferase or a genetically engineered, functional derivative thereof, and the other of the said proteins is either
i) an O⁶-alkylguanine-DNA alkyltransferase, or a genetically engineered derivative thereof, which O⁶-alkylguanine-DNA alkyltransferase exhibits, in comparison to the first protein, a different reactivity against at least one O⁶-alkylguanine-DNA substrate (AGTs with altered reactivity are known in the art, cf. e.g. WO 2004/031404 (PCT/EP03/10859), WO 2004/031405 (PCT/EP03/10889) and WO 2005/085431 (PCT/EP2005/050889), the disclosure of these document being incorporated by reference into the present application especially with respect to AGTs with altered reactivity as disclosed therein); or
ii) ACTs, that specifically react with benzylcytosine BC derivatives and which allow the labelling of ACT fusion proteins using BC derivatives the same way as AGT fusion proteins can be labelled with alkylguanine, especially benzylguanine (hereinafter BG) derivatives, cf. patent application number EP06117779, entitled "Labelling of fusion proteins with synthetic probes", incorporated herein by reference. Importantly, the reactivity of ACT versus BG derivatives is below 1% of the reactivity versus BC derivatives and the reactivity of AGT versus BC derivatives is 1% below the reactivity of AGT versus BG. ACT and AGT thus have substantially non-overlapping substrate selectivity.
iii) a genetically engineered derivative of a hydrolase, in which the hydrolysis step is impaired (such as the above-mentioned HaloTag^{™}).

On the DNA level, the fusion protein can be encoded by DNA selected from the group consisting of genomic DNA, cDNA and recombinant DNA.

In currently preferred embodiments, an expression cassette encoding for a fusion protein as outlined above is provided in an expression vector which is known as such in the art. For example, recombinant DNA encoding for the said fusion protein can be incorporated in any suitable expression vector such as e.g. the pET vectors (Novagen) in which the gene of the fusion protein is under the control of the T7 promoter.

Moreover, the invention provides a prokaryotic or eukaryotic host cell line, enabled to functionally express a fusion protein as outlined above and/or transformed with a an expression vector as outlined above. The cell line can thus be e.g. an insect cell line, a yeast cell line, a bacterial cell line or a mammalian cell line. In specific embodiments, the cell line is a *S. cerevisiae* or an *E. coli* cell line.

Yet a further aspect of the present invention relates to a kit of parts, comprising:
i) a fusion protein comprising at least a first protein and a second protein, wherein both the first and the second proteins are, based on their structure and function, capable of forming a covalent bond with given substrates, and which first and second proteins are of substantially non-overlapping substrate selectivity, preferably of different substrate specificity; and/or
   an expression vector containing an expression cassette encoding for the said fusion protein; and/or
   a prokaryotic or eukaryotic host cell line enabled to functionally express a fusion protein; and
ii) at least one molecule comprising a substrate moiety for at least either the first or the second protein of the said fusion protein.

By providing the molecule comprising a substrate moiety for at least either the first or the second protein of the said fusion protein (lit. ii), above), the user is enabled to individually use the kit-of-parts for his/her specific purpose by reacting the said molecule with his/her object of interest, thereby transferring a substrate moiety for the fusion protein onto the said object. Preferably, the kit-of-parts comprises both a molecule with a substrate moiety for the first protein of the said fusion protein, and a molecule comprising a substrate moiety for the second protein of the said fusion protein. Typical groups that can be used to couple the substrate to the objects are e.g. activated esters that can react with amino, hydroxyl or thiol groups of the objects; maleiimides that can react with thiol groups of the objects; or aldehydes that can react with amino grups of the objects. The person of routine skill in the art will easily choose suitable reactive groups which are able to form a covalent bond under the conditions of the desired application.

In yet further preferred embodiments, the kit-of-parts comprises either a first object already modified with a substrate moiety for the first protein of the said fusion protein; or a second object modified with a substrate moiety for the second protein; or both a first object modified with a substrate moiety for the first protein of the said fusion protein and a second object modified with a substrate moiety for the second protein. Thus, ready-to-use objects may be provided, which proves especially useful for the user e.g. in the case of antibodies, solid surfaces/supports, etc.

Consequently, in yet another aspect of the present invention, a method of crosslinking two objects of interest is provided, comprising the steps of:
i) providing a fusion protein comprising at least a first protein and a second protein, wherein both the first and the second proteins are, based on their structure and function, capable of forming a covalent bond with given substrates, and which first and second proteins are of substantially non-overlapping substrate selectivity, preferably of different substrate specificity;
ii) providing a first object of interest, comprising a substrate moiety for the first protein of the said fusion protein, and providing a second object of interest, comprising a substrate moiety for the second protein of the said fusion protein;
iii) reacting said first protein of the fusion protein with the substrate moiety of said first object, and reacting said second protein of the fusion protein with the substrate moiety of said second object, thereby covalently crosslinking the first object to the second object via the said fusion protein.

The first and the second objects are chosen from the group consisting of spectroscopic probes, affinity handles, receptors, oligonucleotides, solid phases, proteins, enzymes, antibodies, oligonucleotides, DNA, RNA, carbohydrates, lipids, cells, viruses, quantum dots, carbon nanotubes, radioactive molecules, molecules for magnetic resonance imaging, molecules for positron emission tomography, molecules for fluorescence spectroscopy in vitro and in vivo.

In an especially preferred embodiment of the present invention, the above method of crosslinking two objects of interest is used for derivatization of an antibody, wherein either the first object or the second object is an antibody, and the respective other object is chosen from the group consisting of labels, affinity handles, enzymes, proteins, receptors, oligonucleotides, solid phases, antibodies, cells.

In a further particularly useful embodiment of the present invention, the above method of crosslinking two objects of interest is used for derivatization of a cell, wherein either the first object or the second object is a cell, and the respective other object is chosen from the group consisting of labels, affinity handles, receptors, oligonucleotides, solid phases, antibodies, cells.

An additional aspect of the present invention relates to a method of modifying a first object and a second object, for use with a fusion protein comprising at least a first protein and a second protein, wherein both the first and the second protein are, based on their mechanism of enzymatic catalysis, capable of forming a covalent bond with given substrates, and which first and second proteins are of different substrate selectivity, preferably of different substrate specificity, comprising the steps of:
i) transferring to the first object a substrate moiety for the first protein of the said fusion protein; and
ii) transferring to the second object a substrate moiety for the second protein of the said fusion protein.

Of course, the fusion protein of the first and second protein can be designed by genetic engineering to allow for subsequent cleavage by a protease. Towards this end, a protease site can be introduced e.g. in between the first and the second protein; the fusion protein may thus be specifically cleaved again e.g. after crosslinking of the two objects, if desired so, e.g. for control experiments.

## Claims

1. A fusion protein comprising at least a first protein and a second protein, wherein both the first and the second proteins are, based on their structure and function, capable of forming a covalent bond with given substrates, and which first and second proteins are of substantially non-overlapping substrate selectivity, preferably of different substrate specificity; with the provisio that the fusion protein is not ^{M}AGT-DEVD-^{L}AGT or ^{L}AGT-DEVD-^{M}AGT.

2. A fusion protein of claim 1, wherein the first and the second proteins are of orthogonal substrate specificity.

3. A fusion protein of one of claims 1 to 2, wherein one of the said proteins is an O⁶-alkylguanine-DNA alkyltransferase, an alkylcytosine transferase, or a genetically engineered derivative thereof, and wherein the other of the said proteins is either
i) an O⁶-alkylguanine-DNA alkyltransferase, an alkylcytosin transferase, or a genetically engineered derivative thereof; or
ii) a genetically engineered derivative of a hydrolase, in which the hydrolysis step is impaired.

4. A recombinant DNA sequence encoding for a fusion protein of one of claims 1 to 3.

5. An expression vector containing an expression cassette encoding for a fusion protein according to one of claims 1 to 3.

6. A prokaryotic or eukaryotic host cell line, enabled to functionally express a fusion protein according to one of claims 1 to 3 and/or transformed with an expression vector according to claim 5.

7. A kit-of-parts, comprising:
i) a fusion protein comprising at least a first protein and a second protein, wherein both the first and the second proteins are, based on their mechanism of enzymatic catalysis, capable of forming a covalent bond with given substrates, and which first and second proteins are of substantially non-overlapping substrate selectivity, preferably of different substrate specificity; and/or
an expression vector containing an expression cassette encoding for the said fusion protein; and/or
a prokaryotic or eukaryotic host cell line enabled to functionally express a fusion protein; and
ii) at least one molecule comprising a substrate moiety for at least either the first or the second protein of the said fusion protein.

8. A kit-of-parts of claim 7, comprising both a molecule comprising a substrate moiety for the first protein of the said fusion protein, and a molecule comprising a substrate moiety for the second protein of the said fusion protein.

9. A kit-of-parts of one of claims 7 or 8, comprising either a first object modified with a substrate moiety for the first protein of the said fusion protein; or a second object modified with a substrate moiety for the second protein; or both a first object modified with a substrate moiety for the first protein of the said fusion protein and a second object modified with a substrate moiety for the second protein.

10. A method of crosslinking two objects of interest, comprising the steps of:
i) providing a fusion protein comprising at least a first protein and a second protein, wherein both the first and the second protein are, based on their structure and function, capable of forming a covalent bond with given substrates, and which first and second proteins are of substantially non-overlapping substrate selectivity, preferably of different substrate specificity;
ii) providing a first object of interest, comprising a substrate moiety for the first protein of the said fusion protein, and providing a second object of interest, comprising a substrate moiety for the second protein of the said fusion protein;
iii) reacting said first protein of the fusion protein with the substrate moiety of said first object, and reacting said second protein of the fusion protein with the substrate moiety of said second object, thereby covalently crosslinking the first object to the second object via the said fusion protein.

11. A method of claim 10, wherein the first and the second objects are chosen from the group consisting of spectroscopic probes, affinity handles, receptors, oligonucleotides, solid phases, proteins, enzymes, antibodies, oligonucleotides, DNA, RNA, carbohydrates, lipids, cells, viruses, quantum dots, carbon nanotubes, radioactive molecules, molecules for magnetic resonance imaging, molecules for positron emission tomography, molecules for fluorescence spectroscopy.

12. A method of claim 10, for derivatization of an antibody, wherein either the first object or the second object is an antibody, and the respective other object is chosen from the group consisting of spectroscopic probes, affinity handles, receptors, oligonucleotides, solid phases, proteins, enzymes, antibodies, oligonucleotides, DNA, RNA, carbohydrates, lipids, cells, viruses, quantum dots, carbon nanotubes, radioactive molecules, molecules for magnetic resonance imaging, molecules for positron emission tomography, molecules for fluorescence spectroscopy.

13. A method of claim 10, for the crosslinking of proteins, wherein both the first object and the second object are proteins.

14. A method of claim 10, for derivatization of a cell, wherein either the first object or the second object is a cell, and the respective other object is chosen from the group consisting of spectroscopic probes, affinity handles, receptors, oligonucleotides, solid phases, proteins, enzymes, antibodies, oligonucleotides, DNA, RNA, carbohydrates, lipids, cells, viruses, quantum dots, carbon nanotubes, radioactive molecules, molecules for magnetic resonance imaging, molecules for positron emission tomography, molecules for fluorescence spectroscopy.

15. A method of modifying a first object and a second object, for use with a fusion protein comprising at least a first protein and a second protein, wherein both the first and the second protein are, based on their mechanism of enzymatic catalysis, capable of forming a covalent bond with given substrates, and which first and second proteins are of substantially non-overlapping substrate selectivity, preferably of different substrate specificity, comprising the steps of:
i) transferring to the first object a substrate moiety for the first protein of the said fusion protein; and
ii) transferring to the second object a substrate moiety for the second protein of the said fusion protein.


SEQUENCE LISTING

<110>  Ecole Polytechnique Fédérale de Lausanne (EPFL)

<120>  Method of crosslinking two objects of interest

<130>  PEPF002ep

<160>  3

<170>  PatentIn version 3.1

<210>  1
<211>  387
<212>  PRT
<213>  Artificial Sequence

<220>
<223>  fusion protein of human AGTs, each comprising mutations as indica
       ted

<220>
<221>  MISC_FEATURE
<222>  (382)..(387)
<223>  6x histidine tag

<220>
<221>  MISC_FEATURE
<222>  (195)..(381)
<223>  LAGT as described in Heinis et al. ACS Chemical Biology, 1, 575-5
       84 (2006)

<220>
<221>  MISC_FEATURE
<222>  (1)..(182)
<223>  MAGT as described by Juillerat et al. in ChemBioChem, 6, 1263-126
       9 (2005)

<220>
<221>  MISC_FEATURE
<222>  (183)..(194)
<223>  linker, comprising DEVD which is recognized by caspases

<400>  1

<210>  2
<211>  508
<212>  PRT
<213>  Artificial Sequence

<220>
<223>  fusion protein of HaloTag(TM) and human AGT carrying mutations as
        indicated

<220>
<221>  MISC_FEATURE
<222>  (1)..(20)
<223>  preceeding peptide with 6x His tag

<220>
<221>  MISC_FEATURE
<222>  (317)..(326)
<223>  linker containing the recognition site for the preScission Protea
       se (GE Healthcare)

<220>
<221>  MISC_FEATURE
<222>  (327)..(508)
<223>  AGT mutant as described in Gronemeyer et al., Protein Eng. Des. S
       el. 19, 309-318 (2006)


<220>
<221>  MISC_FEATURE
<222>  (21)..(316)
<223>  HaloTag(TM)


<400>  2

Met Gly Ser Ser His His His His His His Ser Ser Gly Leu Val Pro
1               5                   10                  15


Arg Gly Ser His Met Gly Ser Glu Ile Gly Thr Gly Phe Pro Phe Asp
            20                  25                  30


Pro His Tyr Val Glu Val Leu Gly Glu Arg Met His Tyr Val Asp Val
        35                  40                  45


Gly Pro Arg Asp Gly Thr Pro Val Leu Phe Leu His Gly Asn Pro Thr
    50                  55                  60


Ser Ser Tyr Leu Trp Arg Asn Ile Ile Pro His Val Ala Pro Ser His
65                  70                  75                  80


Arg Cys Ile Ala Pro Asp Leu Ile Gly Met Gly Lys Ser Asp Lys Pro
                85                  90                  95


Asp Leu Asp Tyr Phe Phe Asp Asp His Val Arg Tyr Leu Asp Ala Phe
            100                 105                 110


Ile Glu Ala Leu Gly Leu Glu Glu Val Val Leu Val Ile His Asp Trp
        115                 120                 125


Gly Ser Ala Leu Gly Phe His Trp Ala Lys Arg Asn Pro Glu Arg Val
    130                 135                 140


Lys Gly Ile Ala Cys Met Glu Phe Ile Arg Pro Ile Pro Thr Trp Asp
145                 150                 155                 160


Glu Trp Pro Glu Phe Ala Arg Glu Thr Phe Gln Ala Phe Arg Thr Ala
                165                 170                 175

Asp Val Gly Arg Glu Leu Ile Ile Asp Gln Asn Ala Phe Ile Glu Gly
            180                 185                 190

Ala Leu Pro Met Gly Val Val Arg Pro Leu Thr Glu Val Glu Met Asp
        195                 200                 205

His Tyr Arg Glu Pro Phe Leu Lys Pro Val Asp Arg Glu Pro Leu Trp
    210                 215                 220

Arg Phe Pro Asn Glu Leu Pro Ile Ala Gly Glu Pro Ala Asn Ile Val
225                 230                 235                 240

Ala Leu Val Glu Ala Tyr Met Asn Trp Leu His Gln Ser Pro Val Pro
                245                 250                 255

Lys Leu Leu Phe Trp Gly Thr Pro Gly Val Leu Ile Pro Pro Ala Glu
            260                 265                 270

Ala Ala Arg Leu Ala Glu Ser Leu Pro Asn Cys Lys Thr Val Asp Ile
        275                 280                 285

Gly Pro Gly Leu Phe Leu Leu Gln Glu Asp Asn Pro Asp Leu Ile Gly
    290                 295                 300

Ser Glu Ile Ala Arg Trp Leu Pro Gly Leu Ala Gly Leu Glu Val Leu
305                 310                 315                 320

Phe Gln Gly Pro Leu Glu Met Asp Lys Asp Cys Glu Met Lys Arg Thr
                325                 330                 335

Thr Leu Asp Ser Pro Leu Gly Lys Leu Glu Leu Ser Gly Cys Glu Gln
            340                 345                 350

Gly Leu His Glu Ile Ile Phe Leu Gly Lys Gly Thr Ser Ala Ala Asp
        355                 360                 365

Ala Val Glu Val Pro Ala Pro Ala Ala Val Leu Gly Gly Pro Glu Pro
    370                 375                 380

Leu Met Gln Ala Thr Ala Trp Leu Asn Ala Tyr Phe His Gln Pro Glu
385                 390                 395                 400

Ala Ile Glu Glu Phe Pro Val Pro Ala Leu His His Pro Val Phe Gln
                405                 410                 415

Gln Glu Ser Phe Thr Arg Gln Val Leu Trp Lys Leu Leu Lys Val Val
            420                 425                 430

Lys Phe Gly Glu Val Ile Ser Tyr Ser His Leu Ala Ala Leu Ala Gly
        435                 440                 445

Asn Pro Ala Ala Thr Ala Ala Val Lys Thr Ala Leu Ser Gly Asn Pro
    450                 455                 460

Val Pro Ile Leu Ile Pro Cys His Arg Val Val Gln Gly Asp Leu Asp
465                 470                 475                 480

Val Gly Gly Tyr Glu Gly Gly Leu Ala Val Lys Glu Trp Leu Leu Ala
                485                 490                 495

His Glu Gly His Arg Leu Gly Lys Pro Gly Leu Gly
            500                 505

<210>  3
<211>  182
<212>  PRT
<213>  Artificial Sequence

<220>
<223>  engineered AGT (carrying mutations as indicated), converted into
       an ACT

<220>
<221>  MISC_FEATURE
<222>  (1)..(182)
<223>  The sequence is based on an AGT mutant described in Gronemeyer et
        al., Protein Eng. Des. Sel. 19, 309-318 (2006), containing the f
       ollowing additional mutations: Y114R; K131S; S135D; V138E; E159M.

<400>  3

Met Asp Lys Asp Cys Glu Met Lys Arg Thr Thr Leu Asp Ser Pro Leu
1               5                   10                  15

Gly Lys Leu Glu Leu Ser Gly Cys Glu Gln Gly Leu His Glu Ile Ile
            20                  25                  30

Phe Leu Gly Lys Gly Thr Ser Ala Ala Asp Ala Val Glu Val Pro Ala

        35                  40                  45

Pro Ala Ala Val Leu Gly Gly Pro Glu Pro Leu Met Gln Ala Thr Ala
    50                  55                  60

Trp Leu Asn Ala Tyr Phe His Gln Pro Glu Ala Ile Glu Glu Phe Pro
65                  70                  75                  80

Val Pro Ala Leu His His Pro Val Phe Gln Gln Glu Ser Phe Thr Arg
                85                  90                  95

Gln Val Leu Trp Lys Leu Leu Lys Val Val Lys Phe Gly Glu Val Ile
            100                 105                 110

Ser Arg Ser His Leu Ala Ala Leu Ala Gly Asn Pro Ala Ala Thr Ala
        115                 120                 125

Ala Val Ser Thr Ala Leu Asp Gly Asn Pro Val Pro Ile Leu Ile Pro
    130                 135                 140

Cys His Arg Glu Val Gln Gly Asp Leu Asp Val Gly Gly Tyr Met Gly
145                 150                 155                 160

Gly Leu Ala Val Lys Glu Trp Leu Leu Ala His Glu Gly His Arg Leu
                165                 170                 175

Gly Lys Pro Gly Leu Gly
            180